# EUROPEAN PATENT APPLICATION

(11) **EP 1 914 553 A1**
(43) Date of publication of application: **23.04.2008**
(21) Application number: 06122705.4
(22) Date of filing: 20.10.2006
(51) Int. Cl.: G01N 33/68, G01N 33/50, A61P 5/24

(54) **Method for identifying women with an increased risk of foetal growth retardation**

(71) Applicant: THE UNIVERSITY OF NEWCASTLE RESEARCH ASSOCIATES LIMITED, Callaghan, NSW 2308 (AU)
(72) Inventor: Smith, Roger The University of Newcastle, Newcastle New South Wales 2310 (AU)
(74) Representative: Habets, Winand

(57) **Abstract**

The present invention relates to methods for determining or aiding in the determination that a pregnant woman is at risk of developing IUGR or IUGR related disorder(s). In certain embodiments, the invention relates to methods for determining or aiding in the determination that a pregnant woman has IUGR. In further embodiments, the invention relates to methods of screening or pre-screening pregnant women to identify those pregnant women with a low risk of developing IUGR or IUGR related disorders, which reduces the need for additional testing throughout the pregnancy.

## Description

### Field of the invention

This invention relates to methods for the early detection of pregnancy-related disorders by detecting an abnormal level of a number of biomarkers in particular in the serum, plasma or blood of pregnant women. More particularly, the invention relates to a new method for the detection of Intra-Uterine Growth Retardation (IUGR) and babies that are small for their gestational age (SGA), at a relatively early stage of pregnancy.

### Background of the invention

Low birth weight includes two pathologic conditions and one normal condition. The normal condition refers to the healthy but constitutionally small baby which is often referred to as Small for Gestational Age (SGA). The pathologic conditions include preterm delivery and intrauterine growth retardation (IUGR). Synonymous terms found in the literature to describe infants with IUGR include intrauterine growth restriction and foetal growth retardation. In the United States, IUGR is linked to an increase of six to 10 times in perinatal mortality (Bernstein I, Gabbe SG. Intrauterine growth restriction. In: Gabbe SG, Niebyl JR, Simpson JL, Annas GJ, eds. Obstetrics: normal and problem pregnancies. 3d ed. New York: Churchhill-Livingstone, 1996;863-86, . Creasy RK, Resnik R. Intrauterine growth restriction. In: Creasy RK, Resnik R, eds. Maternal-foetal medicine: principles and practice. 3d ed. Philadelphia: Saunders, 1994;558-74).

The current WHO criterion for low birth weight is a weight less than 2,500 g (5 lb, 8 oz) or below the 10th percentile for gestational age (Dunn PM. The search for perinatal definitions and standards. Acta Paediatr Scand Suppl 1985;319:7-16.).

Intrauterine growth retardation (IUGR) is defined as the condition in which foetal growth is delayed or arrested and the actual foetal growth is less than the natural and latent growth corresponding to the gestation age. The condition is caused by foetal, placental and maternal factors, and these three factors coexist in most cases. Concrete causes of IUGR include foetal chromosomal aberration, intrauterine infections and abnormal metabolism, which induce the symmetrical type of IUGR in which the same degree of growth retardation is observed in the head, stature and trunk. Other concrete causes of IUGR include maternal complications such as gestational toxicosis and chronic renal diseases, and disorders of the uteroplacental circulation due to placental or umbilical abnormalities, which induce the asymmetrical type of IUGR in which the growth of head and stature is within normal ranges, while that of trunk is delayed.
IUGR may result in significant foetal morbidity and mortality if not properly diagnosed. When IUGR is recognized, it is important to attempt to correct reversible causes, although many of the conditions responsible for IUGR are not amenable to antenatal therapy. Close foetal surveillance with delivery before 38 weeks of gestation is usually recommended. Some infants born with IUGR have cognitive and medical problems, although for most infants the long-term prognosis is good. However it is now widely accepted that infants born small have an increased likelihood of many adult diseases including early onset hypertension, obesity, diabetes and ischaemic heart disease (Barker, D. J. (1995). "Intrauterine programming of adult disease." Mol Med Today 1 (9): 418-23).

Small for gestational age (SGA) describes an infant's size at birth compared to appropriate population standards and is not synonymous with IUGR which requires antenatal foetal auxology that demonstrates subnormal prenatal growth velocity. Infants born following IUGR may or may not be SGA. The definition of SGA is arbitrary, but in growth clinics SGA is commonly defined as birth weight and/or length two or more standard deviation scores below the mean for gender and gestation, which is consistent with the definition of childhood short stature. As birth length is not always available and is often not as reliably measured as weight, SGA is most commonly defined by birth weight alone.

The known methods for diagnosing the progress of pregnancy, have the disadvantage of detecting the status of pregnancy disorders at a relatively late stage, when the clinical signs and symptoms are already apparent.

Before the development of ultrasonography, delayed foetal growth was indicated by low maternal weight gain, Leopold maneuvers and fundal height measurement. Currently, IUGR is still often suspected on the basis of fundal height measurements. A significant lag in fundal height is a 4 cm or greater difference than expected for gestational age. However, even carefully performed fundal height measurements only have a 26 to 76 percent sensitivity in predicting IUGR (Calvert JP, Crean EE, Newcombe RG, Pearson JF. Antenatal screening measurements by symphysis-fundal height. BMJ 1982; 285: 846-9. 12).

IUGR is frequently detected in a pregnancy with a less-than-expected third-trimester weight gain (100 to 200 g per week) or as an incidental finding on ultrasound examination when foetal measurements are smaller than expected for gestational age.

The main prerequisite for determining IUGR is precise dating. The most accurate dating method uses ultrasound examination at eight to 13 weeks. Later ultrasound examinations are helpful, but the margin of error is increased. The date of the last menstrual period, early uterine sizing and detection of foetal heart tones are helpful ways to accurately date the pregnancy. Most cases of IUGR present during the third trimester, which makes them difficult to accurately diagnose. This is especially true if the patient has presented for prenatal care at a late stage. The physician must determine if the dating is incorrect and the foetal size is actually normal or if the mother truly needs further evaluation for IUGR.

When the suspicion of IUGR is strong, a complete assessment of maternal risk factors is indicated. This includes past medical and obstetric history, medication use, recent infections, occupational or toxic exposures, and a history of tobacco, alcohol or illicit drug use.

Ultrasonography is normally the first study done to assess IUGR. This test loses its accuracy as the pregnancy progresses, but the sensitivity and positive predictive value can be improved if several variables are combined (Doubilet PM, Benson CB. Sonographic evaluation of intrauterine growth retardation. AJR Am J Roentgenol 1995; 164: 709-17). These variables include estimated foetal weight, head circumference and abdominal circumference.

Estimated foetal weight is the most common screen. It is based on the measurements of head circumference, abdominal circumference and femur length. These measurements are plotted on a preexisting standardized chart. In about 95 percent of cases, ultrasound examination allows an estimation of foetal weight with a 15 to 18 percent variance (Doubilet PM, Benson CB. Sonographic evaluation of intrauterine growth retardation, Am. J. Roentgenol. 1995; 164: 709-717). An estimated foetal weight of less than the sixth percentile strongly correlates with growth retardation, and an estimated foetal weight of greater than the 20th percentile virtually rules out IUGR. An estimated foetal weight at the 15th percentile or less, or a decreasing estimated foetal weight as determined by serial ultrasound examination, is suggestive of IUGR.

In all growth-retarded foetuses, the abdominal circumference is the first biometric measure to change. This translates to an increased ratio of head circumference to abdominal circumference. The ratio of head circumference to abdominal circumference is normally one at 32 to 34 weeks and falls below one after 34 weeks. A ratio of greater than one detects about 85 percent of growth-restricted fetuses (Hadlock F. Ultrasound evaluation of foetal growth. In: Callen P, ed. Ultrasonography in obstetrics and gynecology. 3d ed. Philadelphia: Saunders, 1994; 129-42).

There are several hormone assays suggested to give an indication whether placental function is normal or to predict impending foetal death. Among those tests are: urine estriol, urine total estrogens, serum unconjugated estriol and serum placental lactogen.

Estriol is an estrogenic compound produced by the placenta from precursors derived from foetal adrenal cortex and foetal liver. The conjugated form of estriol is excreted into the maternal urine. Serum estriol can be measured either as total estriol or as unconjugated estriol. It usually is measured as unconjugated estriol in order to exclude a maternal contribution to the conjugated fraction. Urine estriol can be measured as total estriol or as total estrogens, since estriol normally constitutes about 90% of urine total estrogens. Estriol can be detected by immunoassay as early as the ninth week of gestation. Thereafter, estriol values slowly but steadily increase until the last trimester, when there is a more pronounced increase. Clinical use of estriol measurement is based on the fact that severe acute abnormality of the fetoplacental unit, such as a dead or dying placenta, is manifested either by failure of the estriol level to continue rising or by a sudden marked and sustained decrease in the estriol level. M.Scharf et.al. (J.Obstet.Gynec.reprod. Biol., 17:365-75, 1984) conclude that in view of the low correlation between patients with abnormal serum free estriol as the antepartum pathological test, the estriol measurement can not be considered a reliable predicting tool to estimate the actual pregnancy outcome.

Urine total estrogen was the first test used, since total estrogen can be assayed by standard clinical techniques. However, urine glucose falsely increases the results and certain other substances such as urobilinogen also may interfere. On the other hand, maternal hypertension, preeclampsia, severe anemia and impaired renal function can decrease considerably urine estrogen or estriol secretion. Decrease in the level may also occur to a variable degree in a number of fetuses with severe congenital anomalies. It was also reported that continued bed-rest to the pregnant woman caused an increase in the estriol excretion values of about 20 to 30% over the levels determined from ambulatory persons. Because of the problems associated with collection of urine or serum estriol specimens and interpretation of the values, as well as the disturbing number of false positive and negative test results, most of the clinical people refrain from correlating these measurements with placental disorders. Moreover, all the previous methods did not reveal the pregnancy disorders at an early stage of their initiation, but only when the particular disease was already apparent.

More recently, another method for the detection of pregnancy disorders including IUGR was disclosed. US Patent 5,198,366 to Silberman describes that abnormal levels of a human placental protein designated PP-13 are indicative for IUGR.

WO99/43851 describes a method for diagnosing susceptibility to IUGR by determining abnormalities in the HLA-G gene, gene expression or protein.

It is an object of the present invention to provide a simple method for the detection of foetal growth retardation at an early stage. It is an another object of the present invention, to provide a simple method for the detection of foetal growth retardation that has a high sensitivity.

### Summary of the invention

The present invention relates to methods for determining or aiding in the determination that a pregnant woman is at risk of developing IUGR or other foetal growth related disorder(s). In certain embodiments, the invention relates to methods for determining or aiding in the determination that a pregnant woman has IUGR, in further embodiments, the invention relates to methods of screening or pre-screening pregnant women to identify those pregnant women with a low risk of developing IUGR or IUGR related disorders, which reduces the need for additional testing throughout the pregnancy.

In more detail, the invention relates to a method for identifying women with an increased risk of IUGR comprising the steps of obtaining a biological sample from a pregnant patient after week 12 and prior to week 37 of pregnancy, in the biological sample, determining the level of at least one biomarker selected from the group consisting of inter-alpha trypsin inhibitor heavy chain H4, antithrombin III, haptoglobin and transthyretin, comparing the level of the at least one biomarker with a level characteristic of normal pregnancies, wherein an abnormal level of said at least one biomarker is indicative for an increased risk of IUGR. In particular, the abnormal level is either a decreased level of inter-alpha trypsin inhibitor heavy chain H4, haptoglobin or transthyretin or an increased level of antithrombin III.

It was surprisingly found that using one of the above methods with the particular biomarker, it is possible to establish an early detection of IUGR. This is in contrast to known prior tests claiming to determine the pregnancy disorders only after their existence in an advanced stage of placental illness.

The invention also relates to a method for ameliorating the effects of a preterm delivery for the newborn, comprising the steps of identifying women with an increased risk of IUGR according to the invention and treating women with an increased risk of IUGR with a steroid or another effective medication in order to promote foetal lung maturation.

The invention also relates to a method for screening or pre-screening pregnant women to identify those pregnant women with a low risk of developing IUGR or IUGR-related disorders, the method comprising the steps of obtaining a biological sample from a pregnant patient after week 12 and prior to week 37 of pregnancy, in the biological sample, determining the level of at least one biomarker selected from the group consisting of inter-alpha trypsin inhibitor heavy chain H4, antithrombin III, haptoglobin and transthyretin, comparing the level of the at least one biomarker with a level characteristic of normal pregnancies wherein a normal level of said at least one biomarker is indicative for a pregnancy with a low risk of developing hypertensive disorders.

### Legend to the figure

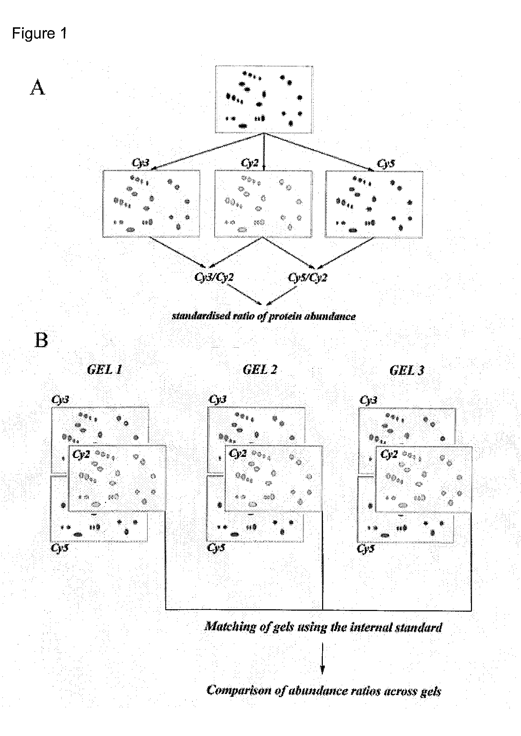

Figure 1. Statistical Analysis of 2D-DIGE Gels. (A) Following imaging, protein spots labelled with either Cy3 or Cy5 are standardised against the pooled internal control spot labelled with Cy2. Gel to gel variation is removed as the samples have all been resolved in the same gel. This is performed in the DIA module of DeCyderTM 2-D Differential Analysis Software. (B) The internal standard is also used for matching and comparing protein spot abundance across gels (performed by the DeCyderTM BVA module). This enables the statistical comparison of protein abundance changes in multiple samples.

### Detailed description of the invention

Birth weight is herein defined as the weight of a baby at its birth. It has direct links with the gestational age at which the child was born and can be estimated during the pregnancy by measuring fundal height. A baby born within the normal range of weight for that gestational age is known as appropriate for gestational age (AGA). Those born above or below that range have often had an unusual rate of development - this often indicates complications with the pregnancy that may affect the baby or its mother.

The incidence of birth weight being outside of the AGA is influenced by the parents in numerous ways, including genetics, the health of the mother, particularly during the pregnancy and environmental factors. There have been numerous studies that have attempted, with varying degrees of success, to show links between birth weight and later-life conditions, including diabetes, obesity, tobacco smoking and intelligence.

Large for gestational age (LGA) is herein defined as babies whose birth weight lies above the 90th percentile for that gestational age. Macrosomia, also known as big baby syndrome, is sometimes used synonymously with LGA, or is otherwise defined as a fetus that weighs above 4000 to 4500 grams regardless of gestational age.

Small for gestational age (SGA) is herein defined as babies whose birth weight lies below the 10th percentile for that gestational age. They may or may not have been the subject of intrauterine growth retardation (IUGR). Low birth weight, is sometimes used synonymously with SGA, or is otherwise defined as a fetus that weighs less than 2500 g regardless of gestational age.

There is a 4-8% incidence of low birth weight in developed countries, and 6-30% in developing countries. Much of this can be attributed to the health of the mother during pregnancy. One third of babies born with a low birth weight are also small for gestational age.

SGA is generally diagnosed by measuring the mother's uterus, with the fundal height being less than it should be for that stage of the pregnancy. If it is suspected, the mother will usually be sent for an ultrasound to confirm.

There are two distinct categories of growth retardation, indicating the stage at which the development was slowed. Small for gestational age babies can be classified as having symmetrical or assymmetrical growth retardation.

Symmetrical growth retardation, less commonly known as global growth retardation, indicates that the fetus has developed slowly throughout the duration of the pregnancy and was thus affected from a very early stage. The head circumference of such a newborn is in proportion to the rest of the body. Common causes include early intrauterine infections, such as cytomegalovirus, rubella or toxoplasmosis, chromosomal abnormalities, maternal substance abuse, such as fetal alcohol spectrum disorder.

Asymmetrical growth retardation occurs when the embryo/fetus has grown normally for the first two trimesters but encounters difficulties in the third, usually pre-eclampsia. Such babies have a disparity in their length and head circumference when compared to the birth weight. A lack of subcutaneous fat leads to a thin and small body out of proportion with the head. Other symptoms include dry, peeling skin and an overly-thin umbilical cord, and the baby is at increased risk of hypoxia and hypoglycaemia.

Possible treatments include the early induction of labour, though this is only done if the condition has been diagnosed and seen as a risk to the health of the fetus.

The present invention relates to a screening assay which provides an early, biochemical indication of increased risk of IUGR. The method can provide an indication of impending IUGR as early as three to four months prior to delivery, such as 2 or 1 month or 4, 3, 2 or 1 week. The method allows early intervention in the course of IUGR and provides an additional factor which can indicate those pregnancies at greatest risk.

The method comprises obtaining a biological sample from the pregnant patient after about week 12 of pregnancy and prior to about week 36 or 37 and determining the level of one or more biomarkers as disclosed herein in the sample. The presence of an abnormal level of the biomarker in the sample indicates a patient who is at risk for IUGR.

In particular, the present invention provides a method for identifying women with an increased risk of IUGR comprising the steps of obtaining a biological sample from a pregnant patient after week 12 and prior to week 37 of pregnancy and in the biological sample, determining the level of at least one biomarker selected from the group consisting of inter-alpha trypsin inhibitor heavy chain H4, antithrombin III, haptoglobin and transthyretin and comparing the level of the at least one biomarker with a level characteristic of pregnancies that proceed to term, wherein an abnormal level of said at least one biomarker is indicative for an increased risk of IUGR.

In contrast to the prior art methods, the method according to the invention is capable of predicting IUGR months in advance, whereas the prior art provides assays that may be used when clinical symptoms of IUGR are already apparent.

IUGR is herein defined as a condition wherein the newborn has a birthweight of less than the 10^{th} percentile combined with a pathological condition that inhibits expression of the normal intrinsic growth potential.

The term abnormal level is meant to indicate either a decreased level of inter-alpha trypsin inhibitor heavy chain H4, haptoglobin or transthyretin or an increased level of antithrombin III.

The term normal pregnancy is meant to indicate a pregnancy that proceeds to term without the complication of IUGR. Preferably, a normal pregnancy is a pregnancy without any other complications, such as in particular preterm delivery, pre-eclampsia or a growth retardation of the foetus. A normal pregnancy as defined herein ends with the delivery of an AGA newborn.

A biological sample may actually be any sample obtained form a pregnant women, the results presented in the present specification are based on sera obtained from pregnant woman. One may also conceive to utilize other biological fluids such as amniotic fluid, vaginal excretions, saliva and urine. Preferably, however, the biological sample is a blood, plasma or serum sample. It is a great advantage when a screening assay can be performed on a blood, serum or plasma sample, since these samples can be taken from a patient with minimal discomfort.

The levels of a particular biomarker provided herein are to be compared with the levels of that same biomarker in the same type of sample taken from a patient, preferably a number of patients that experienced term delivery. Preferably those control samples are from a normal pregnancy of the same gestational period or age as the period or age of the patient screened for the risk of IUGR

For an even higher precision of the method, the samples may be age matched.

It will be understood that the level of markers as disclosed herein can be measured directly or indirectly by a variety of different techniques. However, if the method is to be used as a screening assay, an immunological method is preferred. Antibodies against all the biomarkers provided herein are readily available and a person skilled in the art will know how to design and develop an immunological assay based on such antibodies. For some of the markers, even commercial assays are available.

Particularly preferred immunological methods are radioimmunoassays and enzyme-linked immunosorbent assays (ELISAs) because they are particularly amenable to routine laboratory practice.

A significant problem for premature babies is lung immaturity. Foetal lungs can be matured by the administration of beta methasone. However, if premature delivery is indicated, the beta methasone needs to be administered quickly in order to permit lung maturation to occur prior to delivery.

When IUGR is diagnosed according to a method as described above, premature delivery may be indicated. The chances for survival of the foetus increase when at that point in time effective medication is supplied to promote foetal lung maturation. The invention is therefore also directed to a method for ameliorating the effects of a preterm delivery for the newborn, comprising the steps of identifying women with an increased risk of IUGR according to the invention and treating women with an increased risk of IUGR with a steroid or another effective medication in order to promote foetal lung maturation.

Since the methods according to the invention also have a good negative predictive value, the invention is also directed towards a method for screening or pre-screening pregnant women to identify those pregnant women with a low risk of developing IUGR or IUGR related disorders, the method comprising the steps of obtaining a biological sample from a pregnant patient after week 12 and prior to week 37 of pregnancy, in the biological sample, determining the level of at least one biomarker selected from the group consisting of inter-alpha trypsin inhibitor heavy chain H4, antithrombin III, haptoglobin and transthyretin, comparing the level of the at least one biomarker with a level characteristic of pregnancies that proceed to term, wherein a normal level of said at least one biomarker is indicative for a pregnancy with a low risk of developing hypertensive disorders.

### Patients to be tested

The present method can be used on any pregnant woman following about 12 weeks gestation and prior to term pregnancies (week 36 or 37). In addition to screening any pregnant woman to determine whether the occurrence of IUGR is likely, the patients who are preferably screened are those patients with clinically intact membranes in a high risk category for IUGR.

Any pregnant woman at 12 or more weeks gestation with clinically intact membranes and having one or more risk factors for IUGR is preferably tested throughout the risk period; i.e., until about week 34 to 37.

Preferably, the method is used on samples taken in the second trimester of pregnancy. The levels of the biomarkers disclosed in this application may be measured in blood, preferably in serum or plasma.

Preferably, the samples should be tested immediately after preparation. Storage up to several days under appropriate conditions was shown not to affect the results, nevertheless, addition of protein stabilisers and protease inhibitors such as kallikrein inhibitors (PMSF) is recommended..

### Assay procedures

Abnormal levels of Inter-alpha trypsin Inhibitor heavy chain H4 (ITI H4) were found to be indicative for the diagnosis of IUGR. On average, patients with a normal pregnancy exhibited a level of ITI H4 that was about 2.3 times higher as compared to patients with IUGR.

Levels of ITI H4 were measured using two dimensional differential gel electrophoresis (2D-DIGE) as described in the examples. 2D-DIGE is a powerful tool to enable simultaneous visualization of relatively large portions of the proteome (Marouga et al., Anal. Bioanal. Chem. 2005. 382:669-78). For the routine determination of ITI H4 in the clinical laboratories, an immunological method is preferred. Such methods are further detailed in example 10.

Moreover, methods for the quantitative determination of inter-alpha trypsin inhibitors have been described. EP 0764849 A1 describes an immunological ELISA for the determination of ITI H1 and H2 whereas WO 01/63280 describes the development of a monoclonal antibody specific for ITI.

The molecular cloning of ITI H4 has been described in Saguchi K. Et al., J. Biochem. 119: 898-905 (1996). This allows the expression of recombinant ITI H4 and the generation of monoclonal and polyclonal antibodies against this marker protein. Moreover, methods for the quantitative determination of inter-alpha trypsin inhibitors have been described. EP 0764849 A1 describes an immunological ELISA for the determination of ITI H1 and H2 whereas WO 01/63280 describes the development of a monoclonal antibody specific for ITI. Choi-Miura describes a quantitative ELISA for the measurement of IHRP in human plasma which is believed to be identical with ITI H4 (Biol. Pharm. Bull. 24; 214-217).

Abnormal levels of antithrombin III were also found to be indicative for the diagnosis of IUGR. On average, patients with IUGR exhibited a level of antithrombin III that was about 1.8 times higher as compared to patients with a normal pregnancy.

Levels of antithrombin III were measured using two dimensional differential gel electrophoresis (2D-DIGE) as described in the examples. For the routine determination of antithrombin III in the clinical laboratories, an immunological method is preferred. Such methods are described in example 10.

Mouse monoclonal antibodies against human antithrombin III are commercially available from AntibodyShop A/S in Gentofte, Denmark.

Abnormal levels of haptoglobin were also found to be indicative for the diagnosis of IUGR. On average, patients with IUGR exhibited a level of haptoglobin that was about 1.9 times lower as compared to patients with a normal pregnancy.

Levels of haptoglobin were measured using two dimensional differential gel electrophoresis (2D-DIGE) as described in the examples. For the routine determination of haptoglobin in the clinical laboratories, an immunological method is preferred. Such methods are described in example 10.

Monoclonal and polyclonal antibodies against human haptoglobin are commercially available from AntibodyShop A/S, Grusbakken 8, DK-2820 Gentofte in Denmark and from Genway Biotech Inc in San Diego and from Aviva Systems Biology San Diego.

An ELISA for the sensitive quantitative determination of haptoglobin is commercially available from Gentaur, Av. de l'armee 68, B-1040 Brussels Belgium and from Genway Biotech Inc in San Diego.

Abnormal levels of transthyretin were also found to be indicative for the diagnosis of IUGR. On average, patients with IUGR exhibited a level of transthyretin that was about 1.9 times lower as compared to patients with a normal pregnancy.

Levels of transthyretin were measured using two dimensional differential gel electrophoresis (2D-DIGE) as described in the examples. For the routine determination of transthyretin in the clinical laboratories, an immunological method is preferred. Such methods are described in example 10.

The gene encoding transthyretin has been described by Mita et al., in Biochem. Biophys. Res. Commun. 124:558-564(1984), thus allowing the production of specific antibodies. Moreover, chicken antibodies against human transthyretin are commercially available from Genway Biotech Inc in San Diego and from Aviva Systems Biology San Diego.

An ELISA for the sensitive determination of transthyretin has been described by Vatassery et al., in Clin Chim. Acta, 1991, 197; 19-25.

### Interpretation of results

Current biomarkers for the determination of the risk of IUGR suffer from the drawback of a low sensitivity. The markers identified herein were found to be remarkably sensitive, at an excellent specificity.

When the lowest measured value of the normal (term delivery, non-hypertensive disorder, appropriate for gestational age) samples was taken as the cut-off value, the method employing anti-thrombin III was found to be 100% specific and 100% sensitive (table 3).

The methods employing markers antithrombin III, haptoglobin and transthyretin were found to be very robust; even when the mean value of the normal samples plus or minus two times the standard deviation was taken as the cut off value, an excellent sensitivity was obtained of 100%, 50% and 50% respectively while the specificity remained at 100% (table 2).

The values obtained for inter-alpha trypsin inhibitor heavy chain H4, varied remarkably within the normal (term delivery without hypertension) group as well as within the IUGR group (tables 1 and 2). At an average standardised abundance of 1.84, the standard deviation of 6 normal samples was 0.97. Due to this high variability, the negative predictive value of this biomarker was somewhat lower than the other two markers from the group of 4 markers disclosed herein. Nevertheless, inter-alpha trypsin inhibitor heavy chain H4 is considered a valuable and specific marker for IUGR since the average standardised abundance of the normal delivery group is about 2.3 times higher than the value of the IUGR group. The sensitivity of this marker was 100% at a specificity of 75% when an appropriate cut-off value was chosen.

Antithrombin III was found to be the most robust marker. It exhibited a specificity and sensitivity of 100%, even if the cut-off value was chosen at average normal value plus 2 times the standard deviation (Table 2).

An even more sensitive method could be obtained when several of the markers presented herein were combined, i.e. when combination assays were used. This is completely in line with the aetiology of IUGR as it is usually considered to have multiple causes.

Any combination of two markers chosen from the three markers presented herein, resulted in a method with an increased sensitivity. Hence, the invention relates to a method as described above, wherein at least two biomarkers were used, selected from the group consisting of inter-alpha trypsin inhibitor heavy chain H4, antithrombin III, haptoglobin and transthyretin.

Sensitivity of the method could even be further improved when three or even all four biomarkers were used in a method according to the invention. Even when the cut-off value of the assays was chosen at the average normal value plus or minus two times the standard deviation of the normal population, a combination assay could be created that was 100% specific and 100% specific. Even more importantly, it was found that on average at least two markers were positive for each of the four samples obtained from the IUGR patients (table 2).

A particularly useful method was obtained when markers transthyretin and antithrombin III were combined. Even if the cut-off value was chosen at average normal value plus or minus almost 3 times the standard deviation, this method yielded a sensitivity and specificity of 100%.

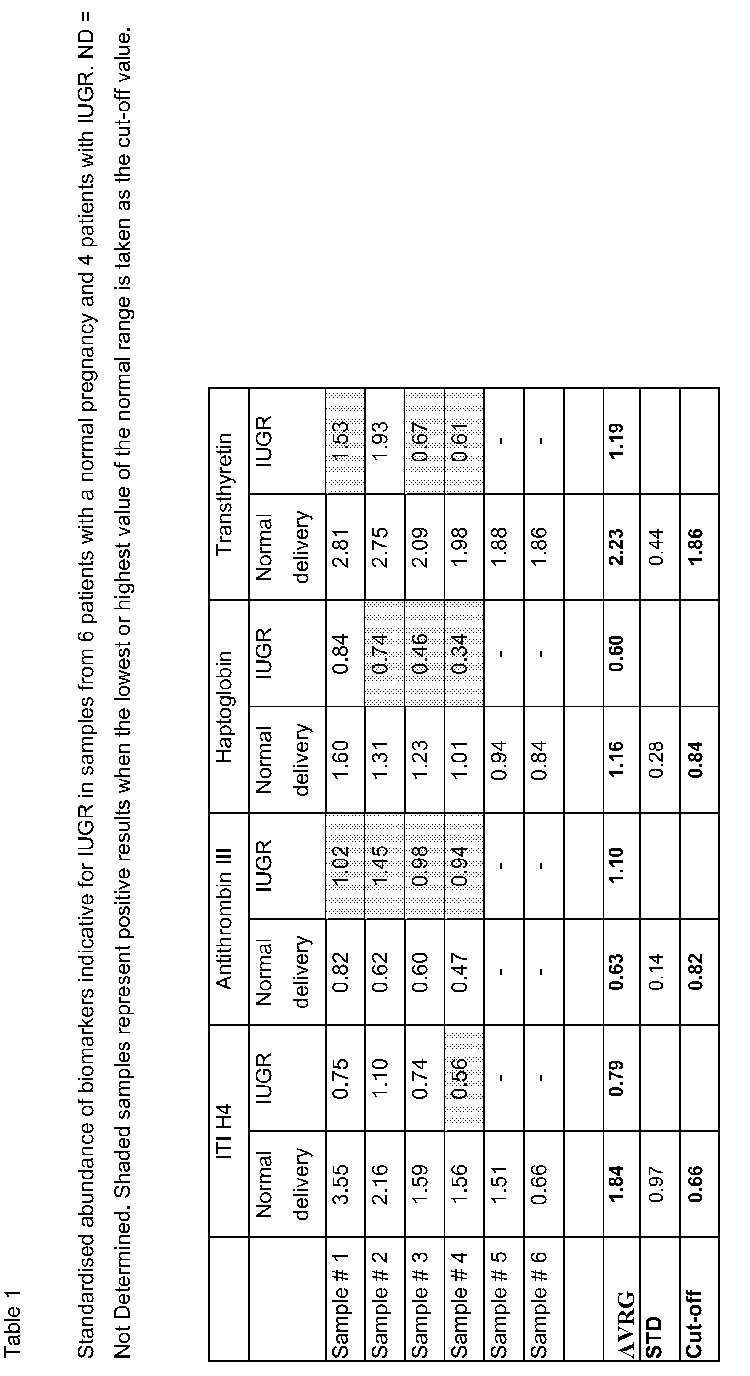

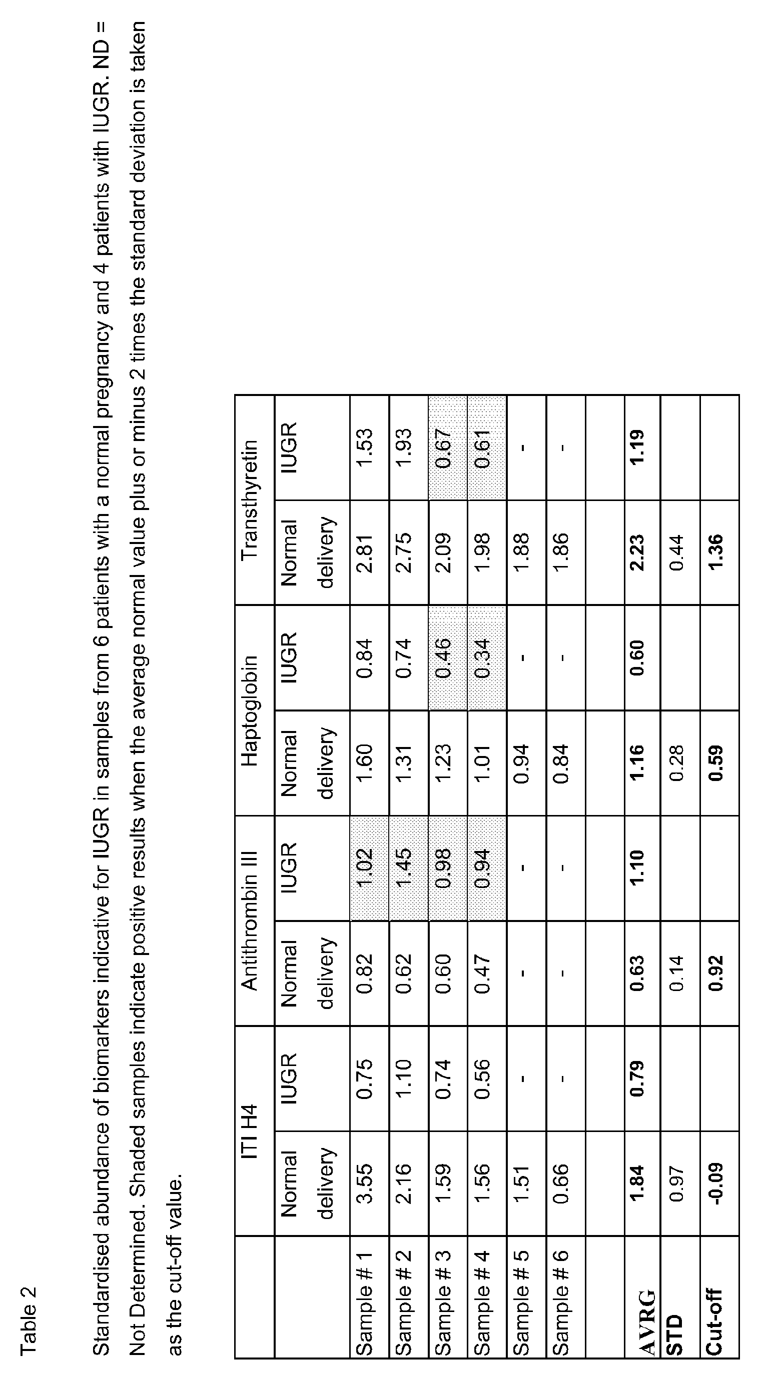

**Table 3.**

| Sensitivity. specificity. positive predictive value and negative predictive value of biomarkers for IUGR. Cut-off value = lowest/highest normal value. | | | | | | | | |
|---|---|---|---|---|---|---|---|---|
| **Biomarker** | **False positive** | **False negative** | **True positive** | **True negative** | **Specificity [%]** | **Sensitivity [%]** | **PPV [%]** | **NPV [%]** |
| ITI H4 | 0 | 3 | 1 | 6 | 100 | 25 | 100 | 67 |
| Antithrombin III | 0 | 0 | 4 | 4 | 100 | 100 | 100 | 100 |
| Haptoglobin | 0 | 1 | 3 | 6 | 100 | 75 | 100 | 86 |
| Transthyretin | 0 | 1 | 3 | 6 | 100 | 75 | 100 | 86 |

### Examples

### Example 1: Plasma samples

Samples were obtained prospectively from pregnant women who were followed until delivery and clinical conditions identified and monitored. Within a cohort of 500 women who experienced IUGR period were identified. A group of normal pregnant women were also identified. Blood plasma samples from these two groups taken at 24 weeks of pregnancy were subjected to two dimensional difference gel electrophoresis (2D-DIGE) analysis. Using 2D-DIGE. the plasma protein profiles of 6 normal pregnant women were compared with plasma proteins profiles from 4 women who experienced IUGR.

### Example 2: Depletion of HSA and IgG and Preparation of Blood Proteins

Human Serum Albumin (HSA) and gamma-immunoglobulin (lgG) account for up to 60% of total blood protein content. This is problematic for protein-based studies of blood because these proteins can mask and prevent the analysis of lower abundance proteins. Samples were depleted of HSA and lgG using the Albumin and lgG Removal Kit (GE Healthcare. Piscataway. NJ. USA) according to the manufacturer's protocol. In brief. extracted samples were incubated with an anti-HSA/anti-lgG resin for 30mins at room temperature. Unbound proteins were separated from the absorption matrix using a mini-spin cartridge and centrifuged at 1000rpm for 2mins. To concentrate protein and remove excess salts. the HSA/IgG depleted samples were precipitated by adding 4 volumes of ice-cold acetone to each sample. The solution was then incubated for 3 hrs at -20°C and then centrifuged at 13000rpm for 15mins at 4°C. Samples were resuspended in 50µl of extraction buffer (7M urea. 2M thiourea. 30mM Tris. 4% w/v CHAPS. pH 8.5). Protein concentration estimation was performed using the 2-D Quant Kit (GE Healthcare).

### Example 3: Fluorescent Cy-dye labelling of samples

Prior to labelling, cyanine dyes Cy2. 3 and 5 (GE Healthcare) were reconstituted in anhydrous DMF. 50µg protein from the normal group and the IUGR group were labelled with 400pM of either Cy3 or Cy5 dye on ice for 30mins in the dark. Half of each sample population was labelled with Cy3 and the other with Cy5 to account for any dye binding bias. An internal pooled sample was created by mixing 25µg of each sample and labelling as above with Cy2. Dye labelling reactions were stopped by adding 10mM lysine and incubating for 10mins on ice in the dark. Cy3 and Cy5-labelled samples were mixed with the Cy2-labelled pooled control and made up to a volume of 450µl with rehydration buffer (7M urea. 2M thiourea. 4% w/v CHAPS. 2mg/mL DTT and 1% v/v pH3-10 immobilised pH gradient (IPG) buffer). See also figure 1.

### Example 4: Two-dimensional gel electrophoresis

In-gel rehydration of IPG strips (non linear. pH3-10. 24cm; GE Healthcare) was performed with the Cy-labelled samples overnight for 12hrs at room temperature under mineral oil (Sigma. St. Louis. MO. USA). Isoelectric focusing was performed using a Multiphor II unit (GE Healthcare) for a total of 58800Vhrs using the following parameters; hold at 300V for 900Vhrs; ramp and hold at 1000V for 3900Vhrs; ramp and hold to 8000V for 13000Vhrs; hold at 8000V for 41000Vhrs. After focusing strips were equilibrated and reduced in equilibration buffer (30% v/v glycerol. 2% w/v SDS. 7M urea. trace bromophenol blue) with 0.5% DTT for 15mins at room temperature and then in equilibration buffer containing 4.5% iodacetamide for 15mins at room temperature. Equilibrated strips were applied on homogeneous 10% acrylamide gels (25.5 x 20.5cm) cast in low-fluorescence plates using the Ettan^{™} DALT*six* Electrophoresis Casting System (GE Healthcare). This enabled the production of six simultaneously-cast gels using the same batch of acrylamide gel stock solution for each gel. Second-dimensional electrophoretic separation was carried out at 2.5W/gel constant power for 30mins then 100W (total) constant power using a peltier-cooled Ettan DALT II electrophoresis unit (GE Healthcare) until the bromophenol dye front reached the bottom of the gel.

### Example 5: Imaging and Analysis of 2D-DIGE gels

Fluorescence image acquisition was performed using the Typhoon 9400 Variable Mode Imager (GE Healthcare). Each gel was scanned at the following excitation/emission wavelengths; 480/530nm (Cy2). 520/590nm (Cy3) and 620/680nm (Cy5). Gel analysis was conducted using the DeCyder V5.0 Biological Variation software module (GE Healthcare). This enabled spot matching between gels and also to quantify and standardise protein spot data. Spot matching was validated by manual inspection of gel spots. Statistically significant protein abundance changes were identified via Student's T-test. Proteins that were present in either all gels or at least in the spontaneous normal labour group with a p-value of p<0.05 or less were chosen as proteins for identification.

### Example 6: Utilisation of the internal standard

The primary advantage of 2D-DIGE over other proteomic techniques is the inclusion of an internal standard control. This allows to compensate for variation within gels and across gels in an experimental series. The internal control was created by pooling equal quantities of all samples to be assessed in each experiment. The pool was labelled with one of the Cy fluorophores (Cy2) and applied to every gel in the experiment containing Cy3 and Cy5 labelled samples. This means that every individual protein spot on the gel was be represented by the Cy2-labelled internal control. Therefore each protein spot could be standardised to its own control spot as well as across all gels in the experiment (Figure 1). In this way gel-to-gel variation was eliminated. This also allowed for the discrimination between system variation and true biological variation.

### Example 7: Protein sequencing

Two sequencing gels were prepared by performing 2D-PAGE on 500µg and 1000µg of pooled myometrium proteins. The gels were fixed in 50% methanol, 7% acetic acid for 30mins at room temperature and then stained with SYPRO Ruby (Invitrogen. California. USA) overnight also at room temperature. The gels were then washed in 10% methanol, 7% acetic acid and briefly rinsed in distilled water before being visualised using a UV illuminator. Proteins of interest were excised from the gels and destained in 50mM ammonium bicarbonate in 50% v/v methanol for 3 washes of 40mins each. Gel plugs were then dried at 37°C for 2hrs and then incubated in 40ng/µl trypsin (Promega, Madison, WI. USA) in 20mM ammonium bicarbonate at 37°C for 3 hours. Protein sequencing was performed via matrix-assisted laser desorption time-of-flight mass spectrometry (MALDI-ToF) using the Ettan MALDI-ToF Pro (GE Healthcare). Approximately 1µl tryptic peptide was mixed with 1µl a-cyano-4-hydroxycinnamic acid matrix (5 mg/ml in 50% acetonitrile, 0.1% trifluroacetic acid) and 1µl of this mixture was spotted in duplicate onto the Maldi target tray. Peptide mass fingerprinting was performed alongside peptide standards (company) in reflectron mode. The external standard was use to calibrate the mass spectrometer prior to acquisition of sample MS data acquisition. Sequence data was used to interrogate the Swiss-Prot and NCBInr databases. As a further control measure, the isoelectric point and molecular weight of positively identified proteins were checked against the region of the pick gel they were excised from.

The following procedures are constructively reduced to practice and therefore described in the present tense. as opposed to the previous examples which were carried out in the laboratory and which were therefore set forth in the past tense.

### Example 8: Purification of polypeptides useful in the Invention

The polypeptide markers that are useful in the invention can be produced by first cloning the corresponding polynucleotides into a mammalian expression vector using established protocols that are familiar to those in skill of the art. In brief, polynucleotides of the invention are amplified and isolated via agarose gel electrophoresis and gel excision. The polynucleotides are subsequently cloned into appropriate vectors to facilitate in-frame cloning. Proteins may for instance be expressed with a polyhistidine metal-binding tag. The vectors with polynucleotides of the invention inserted can then be transfected into a mammalian cell line like COS-7 for high-level expression of the corresponding polypeptides.

The primary nucleotide sequence of all markers useful in the invention may be obtained from Genbank (table 4).

The polypeptides useful in the invention that are produced by the transfected mammalian cell line can then be purified according to established protocols that are familiar to those of skill in the art, for example using the Ni-NTA Magnetic Agarose Beads protein purification protocol, Qiagen, Valencia, Calif. In brief, assays utilizing Ni-NTA Magnetic Beads involve capture of the His-tagged protein-from a cell lysate or a purified-protein solution-followed by washing, binding of interaction partners, further washing, and finally elution of the interacting partner from the still immobilized His-tagged protein or elution of the interacting-partner-His-tagged-protein complex. Between each step, the beads are collected by attracting them to the side of the vessel, after placing near a magnet for 30-60 seconds. Purification procedures may even use crude cell extracts for binding of His-tagged protein.

The resulting purified polypeptides of the invention are then quantified, lyophilized and stored at 4 degrees Celcius or below, and may be used for standards in diagnostic kits or for the generation of antibodies.

### Example 9: Production of Specific Antibodies for the Polypeptides of the Invention

Polyclonal antibodies are produced by DNA vaccination or by injection of peptide antigens into rabbits or other hosts. An animal, such as a rabbit, is immunized with a peptide advantageously conjugated to a carrier protein, such as BSA (bovine serum albumin) or KLH (keyhole limpet hemocyanin). The rabbit is initially immunized with conjugated peptide in complete Freund's adjuvant, followed by a booster shot every two weeks with injections of conjugated peptide in incomplete Freund's adjuvant. Antibodies specific for a polypeptide of the invention are affinity purified from rabbit serum using peptides corresponding to the polypeptides of the invention, for instance coupled to Affi-Gel 10 (Bio-Rad), and stored in phosphate-buffered saline with 0.1 % sodium azide. To determine that the polyclonal antibodies are specific for a polypeptide of the invention, an expression vector encoding a polypeptide of the invention is introduced into mammalian cells. Western blot analysis of protein extracts of non-transfected cells and the cells containing a polypeptide of the invention is performed using the polyclonal antibody sample as the primary antibody and a horseradish peroxidase-labeled anti-rabbit antibody as the secondary antibody. Detection of a band in the cells containing a polypeptide of the invention and lack thereof in the control cells indicates that the polyclonal antibodies are specific for the polypeptide of the invention in question.

Monoclonal antibodies are produced by injecting mice with a peptide derived from a marker polypeptide of the invention, with or without adjuvant. Subsequently, the mouse is boosted every 2 weeks until an appropriate immune response has been identified (typically 1-6 months), at which point the spleen is removed. The spleen is minced to release splenocytes, which are fused (in the presence of polyethylene glycol) with murine myeloma cells. The resulting cells (hybridomas) are grown in culture and selected for antibody production by clonal selection. The antibodies are secreted into the culture supernatant, facilitating the screening process, such as screening by an enzyme-linked immunosorbent assay (ELISA). Alternatively, humanized monoclonal antibodies are produced either by engineering a chimeric murine/human monoclonal antibody in which the murine-specific antibody regions are replaced by the human counterparts and produced in mammalian cells, or by using transgenic "knock out" mice in which the native antibody genes have been replaced by human antibody genes and immunizing the transgenic mice as described above.

Suitable antibodies may also be commercially obtained as described above.

### Example 10: Diagnostic Kit for markers according to the Invention

A diagnostic kit can be made in order to determine the levels of selected polypeptides of the invention that are present in samples obtained from a patient who is undergoing pregnancy. Such samples may be from blood, blood plasma, blood serum, amniotic fluid, saliva or urine.

A diagnostic kit may comprise some or all of the following components: 1) one or more standards comprised of one or more polypeptides of the invention, prepared as described in example 8; 2) an antibody or a plurality of antibodies that are specific for the polypeptides of the invention that are being tested by the kit, prepared as described in example 9; 3) written instructions; 4) diluents for samples and the standards; 5) a wash buffer; 6) color reagents; 7) stop solution; and 8) an antibody carrier such as polystyrene beads or a lateral flow device or a microplate with bound antibody.

An example of such a kit is a quantificative ELISA (enzyme-linked immunosorbent assay) that determines the concentration or concentrations of a polypeptide or polypeptides according to the invention. The principle of the assay is to use the quantitative sandwich enzyme immunoassay technique where a monoclonal or polyclonal antibody selective for binding a polypeptide of the invention is pre-coated onto a carrier such as a microplate into its wells. The standards and sample are then pipetted into the wells and any of the polypeptide of the invention that is present is bound to this immobilized antibody. Next, the wells are washed with washing buffer, and an enzyme-linked monoclonal or polyclonal antibody that is specific for the polypeptide of the invention is added to the wells. Washing again is performed, then a substrate solution is added to the wells. Color subsequently develops in proportion to the amount of polypeptide of the invention that is bound in the first step. The color development is stopped using a stop solution, and the intensity of the color is measured by a microplate reader.

Alternatively, a lateral flow assay may be developed. Such lateral flow assays have the potential to be a cost-effective, fast, simple, and sensitive method, for instance for on-site screening assays. The principle is well known to the skilled person and consist essentially of a carrier that allows a lateral flow to occur wherein either the sample or the detection reagent is displaced form one location on the carrier to another. There are many formats of lateral flow assays suitable for the method according to the invention, the skilled person will know how to choose and optimize a particular format.

### Example 11: Diagnostic Chip for Polypeptides of the Invention

The levels of polypeptides of the invention in biological samples can also be determined through the use of ProteinChip technology (Wright Jr., et al. Expert Review of Molecular Diagnositcs 2002, Vol. 2 pp. 549-63 hereby incorporated herein by reference). This process includes the additon of a few microliters of sample onto the ProteinChip surface, which has both chemical (e.g., anionic, catioinic, hydrophobic, hydrophilic, metal) and biochemical (antibody, receptor, DNA, enzyme probes attached to its surface. Subsequently, binding occurs between the polypeptides of the invention and their respective probes, and the chip is washed at least three times in order to elute unbound protein, lipids, salts, and other substances. Next, an energy-absorbent molecule (EAM) such as sinapinic acid is added to the chip, and the chip is placed into a ProteinChip Reader which measures the molecular mass of the bound polypeptides. Peaks are subsequently produced from the ProteinChip Reader that are used to quantify the amount of polypeptides of the invention in the sample

**Table 4: List of high molecular weight proteins identified as biomarkers of IUGR.**

| **Protein ID** | **Swiss Prot accession # and entry name** | **Synonyms** | **MW of unprocessed precurser (KDa)** | **pl** | **Peptides Sequenced** | **% sequence coverage** |
|---|---|---|---|---|---|---|
| Inter-alphatrypsin heavy chain H4 | Q14624, ITIH4_HUMAN | ITI heavy chain H4, Inter-alpha-inhibitor heavy chain 4, Inter-alpha-trypsin inhibitor family, heavy chain-related protein, IHRP,Plasma kallikrein sensitive glycoprotein 120, PK-120, GP120 | 103.358 | 6.5 | 7 | 8.8 |
| Antithrombin III | P01008 ANT3_HUMAN | ATIII | 52.602 | 6.3 | 6 | 11.9 |
| Haptoglobin | P00738 HPT_HUMAN | | 45.399 | 6.1 | 5 | 10.4 |
| Transthyretin | Q549C7 Q549C7_HUMAN | Prealbumin. TBPA. TTR. ATTR | 15.887 | 5.3 | 6 | 74.4 |

## Claims

1. A method for identifying women with an increased risk of IUGR comprising the steps of
a) Obtaining a biological sample from a pregnant patient after week 12 and prior to week 37 of pregnancy
b) In the biological sample. determining the level of at least one biomarker selected from the group consisting of inter-alpha trypsin inhibitor heavy chain H4, antithrombin III, haptoglobin and transthyretin,
c) Comparing the level of the at least one biomarker with a level characteristic of normal pregnancies,
wherein an abnormal level of said at least one biomarker is indicative for an increased risk of IUGR.

2. A method according to claim 1 wherein the abnormal level is either a decreased level of inter-alpha trypsin inhibitor heavy chain H4, haptoglobin and transthyretin or a decreased level of antithrombin III.

3. A method according to claim 1 or 2 which is an immunological method.

4. A method according to claim 3 which is a radioimmunoassay or an ELISA or a lateral flow assay.

5. A method according to claims 1 - 4 wherein the at least one biomarker is antithrombin III.

6. A method according to claims 1 - 5 wherein the biological sample is blood or plasma or serum.

7. A method for ameliorating the effects of a preterm delivery for the newborn. comprising the steps of identifying women with an increased risk of IUGR according to claims 1 - 6 and treating women with an increased risk of IUGR with a steroid or another effective medication in order to promote foetal lung maturation.

8. A method for screening or pre-screening pregnant women to identify those pregnant women with a low risk of developing IUGR or IUGR related disorders, the method comprising the steps of
a) Obtaining a biological sample from a pregnant patient after week 12 and prior to week 37 of pregnancy
b) In the biological sample. determining the level of at least one biomarker selected from the group consisting of inter-alpha trypsin inhibitor heavy chain H4. antithrombin III. haptoglobin and transthyretin
c) Comparing the level of the at least one biomarker with a level characteristic of a normal pregnancy,
wherein a normal level of said at least one biomarker is indicative for a pregnancy with a low risk of developing hypertensive disorders.
